# FASCICULE DE BREVET EUROPEEN

(11) **EP 0 592 571 B1**
(45) Date de publication et mention de la délivrance du brevet: **12.03.1997**
(21) Numéro de dépôt: 92915085.2
(22) Date de dépôt: 30.06.1992
(51) Int. Cl.: C07D 513/04, A61K 31/54

(54) **DERIVES DE 1,2,4-THIADIAZINO[3,4-b]BENZOTHIAZOLE, LEUR PREPARATION ET LES MEDICAMENTS LES CONTENANT**
1,2,4-THIADIAZINO[3,4-b]BENZOTHIAZOL-DERIVATE, IHRE HERSTELLUNG UND SIE ENTHALTENDE ARZNEIMITTEL
1,2,4-THIADIAZINO[3,4-b]BENZOTHIAZOLE DERIVATIVES, THEIR PREPARATION, AND DRUGS CONTAINING SAME

(30) Priorité: 04.07.1991 FR 9108354
(43) Date de publication de la demande: 20.04.1994
(73) Titulaire: RHONE-POULENC RORER S.A., 92160 Antony (FR)
(72) Inventeur: AUDIAU, François, F-94220 Charenton-le-Pont (FR); JIMONET, Patrick, F-78450 Villepreux (FR); MIGNANI, Serge, F-75014 Paris (FR)
(74) Mandataire: Savina, Jacques
(86) Numéro de dépôt international: FR9200611
(87) Numéro de publication internationale: WO9301194

(56) Documents cités:
- EP-A- 0 374 040
- EP-A- 0 375 510
- EP-A- 0 409 692
- JOURNAL OF ORGANIC CHEMISTRY vol. 44, no. 22, 1979, EASTON US pages 3847 - 3858; J.J. KRUTAK ET AL.: 'Chemistry of Ethenesulfonyl Fluoride. Fluorosulfonylethylation of Organic Compounds' see page 3852, table IV, compound No.46
- CHEMISCHE BERICHTE vol. 100, 1967, WEINHEIM DE pages 2159 - 2163; H. BEECKEN: 'Über die Cycloaddition heterocyclischer N-Sulfinyl-amine an Bicyclo[2.2.1]hepten und Äthoxyacetylen' see page 2160, compound 4

## Description

La présente invention concerne des dérivés de formule : 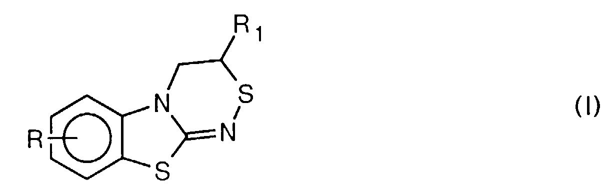 leurs sels, leur préparation et les médicaments les contenant.

Le brevet EP 374040 décrit des dérivés d'imino-2 benzothiazoline actifs vis-à-vis des convulsions induites par le glutamate.

Dans la formule (I),
- R représente un radical polyfluoroalcoxy, polyfluoroalkyle, alcoxy ou alkyle,
- R₁ représente un atome d'hydrogène ou un radical alkyle.

Dans les définitions qui précédent et celles qui seront citées ci-après, les radicaux et les portions alkyle et alcoxy contiennent 1 à 4 atomes de carbone en chaîne droite ou ramifiée.

Les radicaux polyfluoroalkyle sont de préférence les radicaux trifluorométhyle, trifluoro-2,2,2 éthyle et pentafluoroéthyle.

Les radicaux polyfluoroalcoxy sont de préférence les radicaux trifluorométhoxy, pentafluoroéthoxy, trifluoro-2,2,2 éthoxy et tétrafluoro-1,1,2,2 éthoxy.

L'invention concerne également les sels d'addition des composés de formule (I) avec les acides minéraux ou organiques.

Les composés de formule (I) peuvent être préparés par cyclisation d'un dérivé de formule : 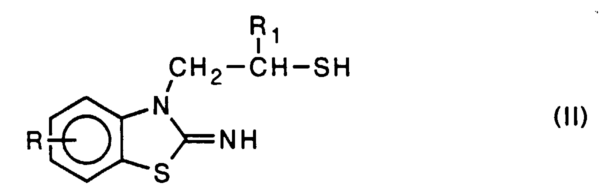 dans laquelle R et R₁ ont les mêmes significations que dans la formule (I).

Cette cyclisation s'effectue généralement en milieu oxydant (brome,chlore, iode, triiodure de potassium, eau oxygénée, chloramine T par exemple) au sein d'un solvant inerte tel qu'un alcool (méthanol, éthanol par exemple), un solvant chloré (chloroforme, chlorure de méthylène par exemple), un solvant aromatique (benzène, toluène par exemple), une cétone (acétone par exemple), à une température variant de 20 à 50°C.

Les dérivés de formule (Il) peuvent être obtenus par hydrolyse d'un dérivé de formule : 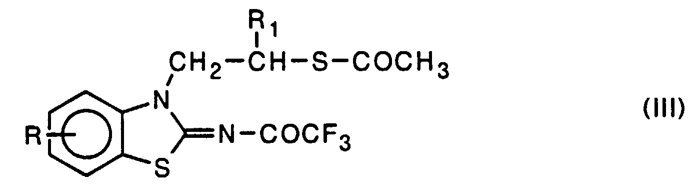 dans laquelle R et R₁ ont les mêmes significations que dans la formule (I).

Cette hydrolyse s'effectue de préférence au moyen d'une base telle qu'un carbonate de métal alcalin (sodium, potassium par exemple), un hydroxyde de métal alcalin (soude, potasse par exemple), au sein d'un solvant inerte tel qu'un alcool, une cétone, l'eau ou un mélange de ces solvants, à une température comprise entre 20 et 50°C.

Les dérivés de formule (III) peuvent être obtenus par action d'acide thioacétique sur un dérivé de formule : 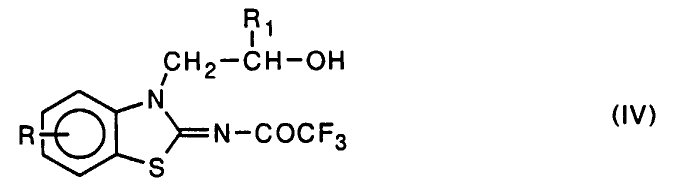 dans laquelle R et R₁ ont les mêmes significations que dans la formule (I).

Cette réaction s'effectue de préférence au sein d'un solvant inerte tel que le tétrahydrofuranne, au moyen de triphénylphosphine et d'azodicarboxylate d'éthyle, à une température comprise entre 0 et 25°C.

Les dérivés de formule (IV) peuvent être préparés par application ou adaptation de la méthode décrite dans le brevet EP409692.

Les mélanges réactionnels obtenus par les divers procédés décrits précédemment sont traités suivant des méthodes classiques physiques (évaporation, extraction, distillation, chromatographie, cristallisation....) ou chimiques (formation de sels....).

Les composés de formule (I) sous forme de base libre peuvent être éventuellement transformés en sels d'addition avec un acide minéral ou organique, par action d'un tel acide au sein d'un solvant organique tel qu'un alcool, une cétone, un éther ou un solvant chloré.

Les composés de formule (I) et leurs sels présentent des propriétés pharmacologiques intéressantes. Ces composés interfèrent avec la transmission glutamatergique et sont donc utiles dans le traitement et la prévention des phénomènes liés au glutamate. Ces phénomènes sont notamment les manifestations épileptogènes et/ou convulsives, les troubles schizophréniques et en particulier les formes déficitaires de la schizophrénie, les troubles du sommeil, l'anxiété, les phénomènes liés à l'ischémie cérébrale ainsi que les troubles neurologiques liés au vieillissement où le glutamate peut être impliqué telles que la maladie d'Alzheimer, la maladie d'Huntington, la sclérose latérale amyotrophique et l'atrophie olivopontocérébelleuse.

L'activité de ces produits comme antiglutamate a été déterminée sur les convulsions induites par le glutamate selon une technique inspirée de celle de 1. P. LAPIN, J. Neural. Transmission, 54, 229-238 (1982); l'injection du glutamate par voie intracérébroventriculaire étant effectuée selon une technique inspirée de celle de R. CHERMAT et P. SIMON, J. Pharmacol. (Paris), 6, 489-492 (1975). Leur DE₅₀ est inférieure ou égale à 10 mg/kg.

Les composés de formule (I) présentent une toxicité faible. Leur DL₅₀ est supérieure à 15 mg/kg par voie IP chez la souris.

Pour l'emploi médicinal, il peut être fait usage des composés de formule (I) tels quels ou à l'état de sels pharmaceutiquement acceptables, c'est-à-dire non toxiques aux doses d'utilisation.

Comme exemples de sels pharmaceutiquement acceptables, peuvent être cités les sels d'addition avec les acides minéraux ou organiques tels que acétate, propionate, succinate, benzoate, fumarate, maléate, oxalate, méthanesulfonate, iséthionate, théophyllinacétate, salicylate, méthylène-bis-β-oxynaphtoate, chlorhydrate, sulfate, nitrate et phosphate.

L'exemple suivant, donné à titre non limitatif, montre comment l'invention peut être mise en pratique.

### EXEMPLE 1

A 1,2 g de trihydrate de chloramine T (Prolabo) en solution dans 15 cm³ de méthanol, on ajoute progressivement 1,2 g de 2-imino-3-(2-mercaptoéthyl)-6-trifluorométhoxy-benzothiazoline à une température voisine de 20°C. La réaction est poursuivie 2 heures à cette température. Le méthanol est évaporé sous pression réduite et le résidu blanchâtre repris dans 50 cm³ d'eau distillée. La phase organique est extraite par 2 fois 30 cm³ d'éther éthylique, lavée à l'eau distillée puis séchée sur sulfate de magnésium. Après filtration et concentration à sec sous pression réduite, on obtient 1,2 g d'une mousse blanche purifiée par flash-chromatographie sur silice avec un mélange acétate d'éthyle-cyclohexane (40-60 en volumes) comme éluant. 0,9 g de 3,4-dihydro-8-trifluorométhoxy-1,2,4-thiadiazino[3,4-b]benzothiazole sont obtenus sous forme d'une huile incolore qui par addition d'acide oxalique dans l'acétone, donne 0,8g d'oxalate fondant à 178°C.

La 2-imino-3-(2-mercaptoéthyl)-6-trifluorométhoxy-benzothiazoline peut être préparée de la façon suivante : à 15,1 g de 3-(2-acétylthioéthyl)-2-trifluoroacétylimino-6-trifluorométhoxy-benzothiazoline en solution dans 500 cm³ de méthanol, on ajoute 75 cm³ d'une solution de carbonate de potassium à 7% dans l'eau à une température voisine de 20°C. Après 72 heures de réaction à cette température, le méthanol est évaporé sous pression réduite et le résidu solide repris dans 500 cm³ d'eau distillée. L'insoluble blanc est filtré, lavé à l'eau puis séché. Après purification par flash-chromatographie sur colonne de silice avec de l'acétate d'éthyle comme éluant, on obtient 6,3 g de 2-imino-3-(2-mercaptoéthyl)-6-trifluorométhoxy-benzothiazoline sous forme de solide blanc fondant à 110°C.

La 3-(2-acétylthioéthyl)-2-trifluoroacétylimino-6-trifluorométhoxy-benzothiazoline peut être obtenue de la manière suivante : dans un ballon placé sous azote, on introduit 21 g de triphénylphosphine et 150 cm³ de tétrahydrofurane. A ce mélange agité à 0-5°C, on ajoute, goutte à goutte, en environ 30 minutes, une solution de 12,8 cm³ d'azodicarboxylate d'éthyle dans 100 cm³ de tétrahydrofurane. L'agitation est poursuivie 1 heure à cette température. Une solution de 14,2 g de 3-(2-hydroxyéthyl)-2-trifluoroacétylimino-6-trifluorométhoxy-benzothiazoline et de 5,6 cm³ d'acide thioacétique dans 250 cm³ de tétrahydrofurane est alors ajoutée goutte à goutte en environ 30 minutes à 0°C. Cette température est maintenue 1 heure puis la réaction poursuivie 15 heures à une température voisine de 20°C. Après concentration à sec sous pression réduite, le résidu est purifié par flash-chromatographie sur colonne de silice avec un mélange cyclohexane-acétate d'éthyle (95-5 en volumes) comme éluant. On obtient ainsi 15,3 g de produit attendu sous forme d'une poudre blanche fondant à 120°C.

La 3-(2-hydroxyéthyl)-2-trifluoroacétylimino-6-trifluorométhoxy-benzothiazoline peut être préparée selon la méthode décrite dans le brevet EP 409692.

Les médicaments selon l'invention sont constitués par un composé de formule (I) sous forme libre ou sous forme d'un sel d'addition avec un acide pharmaceutiquement acceptable, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, intraveineuse, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vemis.

Comme compositions liquides pour administration orale, on peut utiliser des solutions, des suspensions, des émulsions, des sirops et des élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

En thérapeutique humaine, les composés selon l'invention interfèrent avec la transmission glutamatergique et sont donc particulièrement utiles dans le traitement et la prévention des désordres liés au glutamate. Ces composés sont notamment utiles pour le traitement ou la prévention des manifestations épileptogènes et/ou convulsives, des troubles schizophréniques et notamment des formes déficitaires de la schizophrénie, des troubles du sommeil, de l'anxiété, des phénomènes liés à l'ischémie cérébrale ainsi que des troubles neurologiques liés au vieillissement où le glutamate peut être impliqué telles que la maladie d'Alzheimer, la maladie d'Huntington, la sclérose latérale amyotrophique et l'atrophie olivopontocérébelleuse.

Les doses dépendent de l'effet recherché, de la durée du traitement et de la voie d'administration utilisée; elles sont généralement comprises entre 30 et 300 mg par jour par voie orale pour un adulte avec des doses unitaires allant de 10 mg à 100 mg de substance active.

D'une façon générale, le médecin déterminera la posologie appropriée en fonction de l'âge, du poids et de tous les autres facteurs propres au sujet à traiter.

Les exemples suivants illustrent des compositions selon l'invention :

### EXEMPLE A

On prépare, selon la technique habituelle, des gélules dosées à 50 mg de produit actif ayant la composition suivante :
- 3,4-dihydro-8-trifluorométhoxy-1,2,4-thiadiazino[3,4-b] benzothiazole 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle de comprimés dosés à 50 mg de produit actif ayant la composition suivante :
- 3,4-dihydro-8-trifluorométhoxy-1,2,4-thiadiazino[3,4-b] benzothiazole 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- 3,4-dihydro-8-trifluorométhoxy-1,2,4-thiadiazino[3,4-b] benzothiazole 10 mg
- Acide benzoïque 80 mg
- Alcool benzylique 0,06 cm3
- Benzoate de sodium 80 mg
- Ethanol à 95 % 0,4 cm3
- Hydroxyde de sodium 24 mg
- Propylène glycol 1,6 cm3
- Eau q.s.p. 4 cm3

## Revendications (Revendications pour l'(les) Etat(s) contractant(s) suivant(s): AT, BE, CH, DE, DK, FR, GB, IT, LI, LU, NL, PT, SE)

1. Composés de formule : 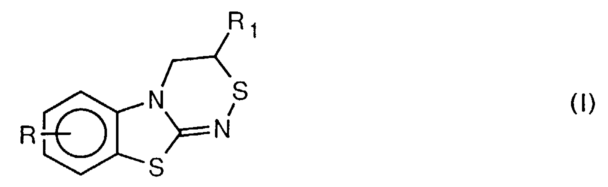 dans laquelle R représente un radical polyfluoroalcoxy, polyfluoroalkyle, alcoxy ou alkyle et R₁ représente un atome d'hydrogène ou un radical alkyle et leurs sels avec un acide minéral ou organique, étant entendu que les radicaux et portions alkyle et alcoxy contiennent 1 à 4 atomes de carbone en chaîne droite ou ramifiée.

2. Composés de formule (I) selon la revendication 1 pour lesquels R représente un radical trifluorométhyle, trifluoro-2,2,2 éthyle, trifluorométhoxy, pentafluoroéthyle, trifluoro-2,2,2 éthoxy ou tétrafluoro-1,1,2,2 éthoxy.

3. Le 3,4-dihydro-8-trifluorométhoxy-1,2,4-thiadiazino[3,4-b]benzothiazole et ses sels avec un acide minéral ou organique.

4. Procédé de préparation des composés de formule (I) selon la revendication 1 caractérisé en ce que l'on cyclise un dérivé de formule : 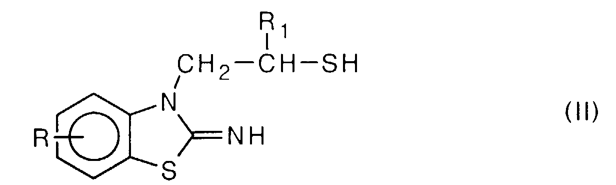 dans laquelle R et R₁ ont les mêmes significations que dans la revendication 1.

5. Médicaments caractérisés en ce qu'ils contiennent en tant que principe actif au moins un composé de formule (I) selon la revendication 1 ou un sel d'un tel composé.

6. Médicaments contenant en tant que principe actif le 3,4-dihydro-8-trifluorométhoxy-1,2,4-thiadiazino[3,4-b]benzothiazole ou un sel de ce composé.

7. Médicaments selon l'une des revendications 5 et 6 qui interfèrent avec la transmission glutamatergique.

## Revendications (Revendications pour l'(les) Etat(s) contractant(s) suivant(s): ES, GR)

1. Procédé de préparation des composés de formule : 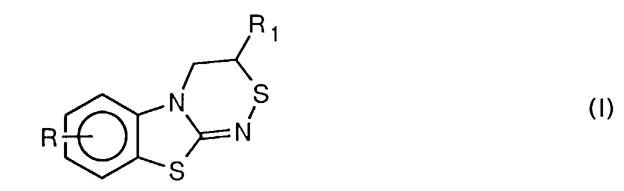 dans laquelle R représente un radical polyfluoroalcoxy, polyfluoroalkyle, alcoxy ou alkyle et R₁ représente un atome d'hydrogène ou un radical alkyle et leurs sels avec un acide minéral ou organique, étant entendu que les radicaux et portions alkyle et alcoxy contiennent 1 à 4 atomes de carbone en chaîne droite ou ramifiée, caractérisé en ce que l'on cyclise un dérivé de formule : 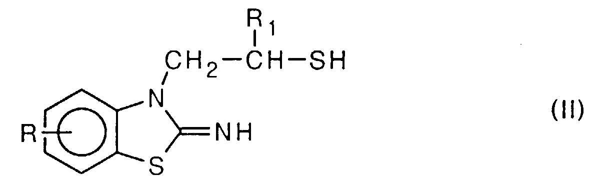 dans laquelle R et R₁ ont les mêmes significations que dans la formule (I), isole le produit et le transforme éventuellement en sel.

2. Procédé selon la revendication 1 pour la préparation des composés de formule (I) pour lesquels R représente un radical trifluorométhyle, trifluoro2,2,2 éthyle, trifluorométhoxy, pentafluoroéthyle, trifluoro-2,2,2 éthoxy ou tétrafluoro-1,1,2,2 éthoxy.

3. Procédé selon la revendication 1 pour la préparation du 3,4-dihydro-8-trifluorométhoxy-1,2,4-thiadiazino[3,4-b]benzothiazole et ses sels avec un acide minéral ou organique.

## Patentansprüche (Patentansprüche für folgende(n) Vertragsstaat(en): AT, BE, CH, DE, DK, FR, GB, IT, LI, LU, NL, PT, SE)

1. Verbindungen der Formel: 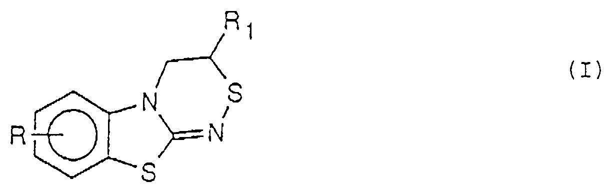 in welcher R für einen Polyfluoralkoxy-, Polyfluoralkyl-, Alkoxy- oder Alkylrest steht und R₁ ein Wasserstoffatom oder einen Alkylrest darstellt, und deren Salze mit einer Mineral- oder organischen Säure, wobei die Alkyl- und Alkoxyreste und -teile 1 bis 4 Kohlenstoffatome in einer geraden oder verzweigten Kette aufweisen sollen.

2. Verbindungen der Formel (I) nach Anspruch 1, für die R einen Trifluormethyl-, 2,2,2-Trifluorethyl-, Trifluor-methoxy-, Pentafluorethyl-, 2,2,2-Trifluorethoxy- oder 1,1,2,2-Tetrafluorethoxyrest darstellt.

3. 3,4-Dihydro-8-trifluormethoxy-1,2,4-thiadiazino[3,4-b]benzothiazol und dessen Salze mit einer Mineral- oder organischen Säure.

4. Verfahren zur Herstellung der Verbindungen der Formel (I) nach Anspruch 1, dadurch gekennzeichnet, daß man ein Derivat der Formel: 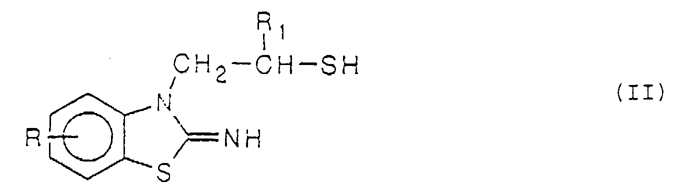 in welcher R und R₁ dieselben Bedeutungen wie im Anspruch 1 aufweisen, cyclisiert.

5. Medikamente, dadurch gekennzeichnet, daß sie als Wirkstoff mindestens eine Verbindung der Formel (I) nach Anspruch 1 oder ein Salz einer derartigen Verbindung enthalten.

6. Medikamente, enthaltend als Wirkstoff 3,4-Dihydro-8-trifluormethoxy-1,2,4-thiadiazino [3,4-b] benzothiazol oder ein Salz dieser Verbindung.

7. Medikamente nach einem der Ansprüche 5 und 6, die mit der Glutamat-Übertragung interferieren.

## Patentansprüche (Patentansprüche für folgende(n) Vertragsstaat(en): ES, GR)

1. Verfahren zur Herstellung von Verbindungen der Formel: 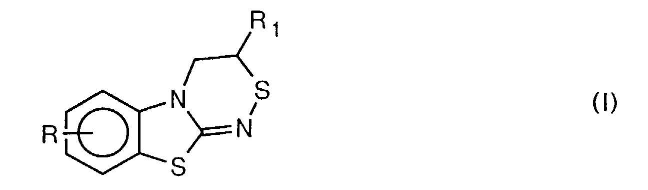 in welcher R für einen Polyfluoralkoxy-, Polyfluoralkyl-, Alkoxy- oder Alkylrest steht und R₁ ein Wasserstoffatom oder einen Alkylrest darstellt, und deren Salze mit einer Mineral- oder organischen Säure, wobei die Alkyl- und Alkoxyreste und -teile 1 bis 4 Kohlenstoffatome in einer geraden oder verzweigten Kette aufweisen sollen, dadurch gekennzeichnet, daß man ein Derivat der Formel: 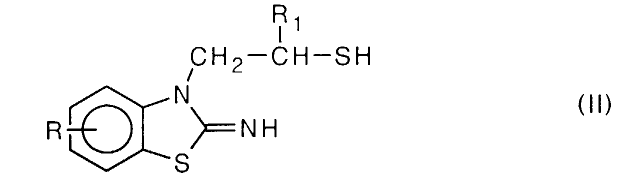 in welcher R und R₁ dieselben Bedeutungen wie in Formel (I) aufweisen, cyclisiert, das Produkt isoliert und es gegebenenfalls in ein Salz überführt.

2. Verfahren nach Anspruch 1 für die Herstellung der Verbindungen der Formel (I), für welche R einen Trifluormethyl-, 2,2,2-Trifluorethyl-, Trifluormethoxy-, Pentafluorethyl-, 2,2,2-Trifluorethoxy- oder 1,1,2,2-Tetrafluorethoxyrest darstellt.

3. Verfahren nach Anspruch 1 für die Herstellung von 3,4-Dihydro-8-trifluormethoxy-1,2,4-thiadiazino[3,4-b]benzothiazol und dessen Salzen mit einer Mineral- oder organischen Säure.

## Claims (Claims for the following Contracting State(s): AT, BE, CH, DE, DK, FR, GB, IT, LI, LU, NL, PT, SE)

1. Compounds of formula 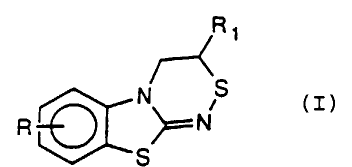 in which R represents a polyfluoroalkoxy, polyfluoroalkyl, alkoxy or alkyl radical, and R₁ represents a hydrogen atom or an alkyl radical, and their salts with an inorganic or organic acid, it being understood that the alkyl and alkoxy radicals and portions contain 1 to 4 carbon atoms in a linear or branched chain.

2. Compounds of formula (I) according to Claim 1, for which R represents a trifluoromethyl, 2,2,2-trifluoroethyl, trifluoromethoxy, pentafluoroethyl, 2,2,2-trifluoroethoxy or 1,1,2,2-tetrafluoroethoxy radical.

3. 3,4-Dihydro-8-trifluoromethoxy-1,2,4-thiadiazino[3,4-b]benzothiazole and its salts with an inorganic or organic acid.

4. Method of preparing compounds of formula (I) according to Claim 1, characterised in that a derivative of formula: 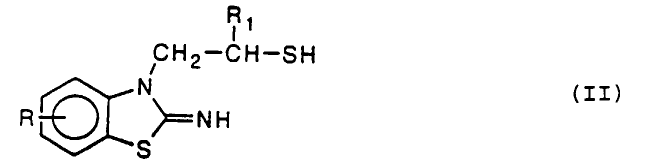 in which R and R₁ have the same meanings as in Claim 1, is cyclised.

5. Medicinal products characterised in that they contain as active ingredient at least one compound of formula (I) according to claim 1, or a salt of such a compound.

6. Medicinal products containing as active ingredient 3,4-dihydro-8-trifluoromethoxy-1,2,4-thiadiazino[3,4-b]benzothiazole or a salt of this compound.

7. Medicinal compounds according to one of Claims 5 and 6 which interfere with glutamatergic transmission.

## Claims (Claims for the following Contracting State(s): ES, GR)

1. Method of preparing compounds of formula: 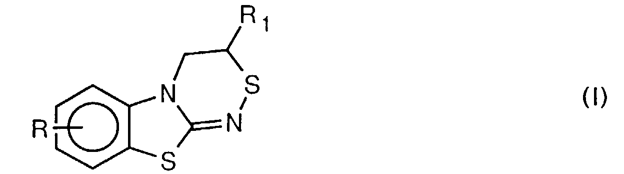 in which R represents a polyfluoroalkoxy, polyfluoroalkyl, alkoxy or alkyl radical, and R₁ represents a hydrogen atom or an alkyl radical, and their salts with an inorganic or organic acid, it being understood that the alkyl and alkoxy radicals and portions contain 1 to 4 carbon atoms in a linear or branched chain, characterised in that a derivative of formula: 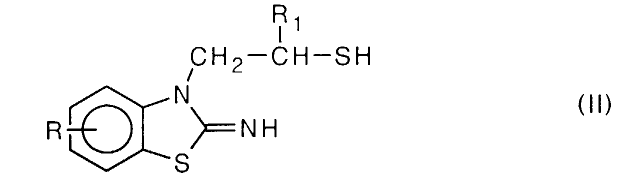 in which R and R₁ have the same meanings as in formula (I), is cyclised, the product is isolated and optionally converted to a salt.

2. Method according to Claim 1, for preparing compounds of formula (I) for which R represents a trifluoromethyl, 2,2,2-trifluoroethyl, trifluoromethoxy, pentafluoroethyl, 2,2,2-trifluoroethoxy or 1,1,2,2-tetrafluoroethoxy radical.

3. Method according to Claim 1, for preparing 3,4-dihydro-8-trifluoromethoxy-1,2,4-thiadiazino[3,4-b] - benzothiazole and its salts with an inorganic or organic acid.
